# EUROPEAN PATENT APPLICATION

(11) **EP 3 078 381 A1**
(43) Date of publication of application: **12.10.2016**
(21) Application number: 15001035.3
(22) Date of filing: 10.04.2015
(51) Int. Cl.: A61K 36/77, A61P 25/28

(54) **HORSE CHESTNUT EXTRACT OR ESCINS FOR THE TREATMENT OF ALZHEIMER'S DISEASE**

(71) Applicant: Warszawski Uniwersytet Medyczny, 02-091 Warszawa (PL); INSTYTUT FARMACEUTYCZNY, 01-793 Warszawa (PL); Instytut Biochemii I Biofizyki Polskiej Akademii Nauk, 02-106 Warszawa (PL)
(72) Inventor: Koziak, Katarzyna, 02-708 Warszawa (PL); Kowalewska, Magdalena, 03-937 Warszawa (PL); Maciejko, Dorota, 02-515 Warszawa (PL); Zegrocka-Stendel, Oliwia, 05-092 Lomianki (PL); Domanski, Dominik, 02-390 Warszawa (PL); Perzanowska, Anna, 05-300 Minsk Mazowiecki (PL)
(74) Representative: Walkiewicz, Sebastian Rafal

(57) **Abstract**

The subject invention relates to agents for use in treatment and/or prevention of Alzheimer's disease. The group of agents include horse chestnut extract, escins, their salts and derivatives.

## Description

### Field of the invention

The subject invention relates to agents for use in treatment and/or prevention of Alzheimer's disease. The group of agents include horse chestnut extract, escin and its salts.

### Prior art

Alzheimer's disease (AD), accounts for 60% to 70% of cases of dementia (Burns A and Iliffe S. "Alzheimer's disease", BMJ 2009; 338:b158; "Dementia Fact sheet N°362". who.int. April 2012). It is a chronic neurodegenerative disease that usually starts slowly and gets worse over time. The most common early symptom is difficulty in remembering recent events (short-term memory loss). As the disease advances, symptoms can include: problems with language, disorientation (including easily getting lost), mood swings, loss of motivation, not managing self care, and behavioral issues. As the condition declines, the patients often withdraw from their families and society. Gradually, bodily functions are lost, ultimately leading to death. The rates of progression can vary and the average life expectancy following diagnosis is three to nine years (Querfurth HW1, LaFerla FM. Alzheimer's disease. N Engl J Med. 2010;362(4):329-44.; Todd S, Barr S, Roberts M, Passmore AP. Survival in dementia and predictors of mortality: a review. Int J Geriatr Psychiatry. 2013;28(11):1109-24).

The cause of Alzheimer's disease is poorly understood. There are several competing hypotheses trying to explain the cause of the disease. The main notion is that extracellular amyloid beta (Aβ) deposits are the fundamental cause of the disease (Hardy J1, Allsop D. Amyloid deposition as the central event in the aetiology of Alzheimer's disease. Trends Pharmacol Sci. 1991;12(10):383-8; Mudher A, Lovestone S, Alzheimer's disease-do tauists and baptists finally shake hands?, Trends in Neurosciences. 2002;25(1):22-26).

Proteolytic processing of amyloid precursor protein (APP) by the transmembrane aspartyl protease p-site APP cleaving enzyme 1 (BACE1) initiates Ap production, a key step in Alzheimer's disease pathogenesis (Vassar et al., Beta-secretase cleavage of Alzheimer's amyloid precursor protein by the transmembrane aspartic protease BACE, Science. 1999;286(5440):735-41.; Yan et al. Membrane-anchored aspartyl protease with Alzheimer's disease beta-secretase activity, Nature. 1999;402(6761):533-7.). It has been also found that a single amino acid substitution adjacent to the BACE1 cleavage site of APP, which significantly reduces BACE1 cleavage and thus Ap peptide generation in cultured cells, protects against disease onset as well as cognitive decline in the elderly without Alzheimer's disease (Jonsson et al. A mutation in APP protects against Alzheimer's disease and age-related cognitive decline, Nature. 2012 Aug 2;488(7409):96-9.). Abnormal accumulation of BACE1 in axon terminals has been documented in Alzheimer's disease brain (Zhao et al. Beta-site amyloid precursor protein cleaving enzyme 1 levels become elevated in neurons around amyloid plaques: implications for Alzheimer's disease pathogenesis, J Neurosci. 2007;27(14):3639-49.).

Although the importance of APP in Alzheimer's disease is indicated by its genetic, biochemical and neuropathological connections to the disease, it has to be stressed that the molecule is a component of acute-phase responses to injury and plays a beneficial role in coping with cellular injury and stress. Moreover, elevation of βAPP expression can be neuroprotective *in vivo* (Masliah E, et al. Amyloid precursor proteins protect neurons of transgenic mice against acute and chronic excitotoxic injuries in vivo, Neuroscience. 1997;78:135-46). It has been described that a progressive increase in neuronal APP expression in the brains of non-demented individuals is associated with increasing age is, whereas in AD patient samples APP antigenicity decreased in neuronal somata in a manner that correlated with accumulation of mature Aβ plaques (Barger SW et al. Relationships Between Expression of Apolipoprotein E and β-Amyloid Precursor Protein Are Altered in Proximity to Alzheimer β-Amyloid Plaques: Potential Explanations from Cell Culture Studies, J Neuropathol Exp Neurol. 2008; 67(8): 773-783.).

Recent studies focused on characterizing the dynamics of endocytic BACE1 trafficking in hippocampal neurons revealed that BACE1 internalized in dendrites is exclusively transported toward the soma, and this polarized transport requires the function of Eps15 homology domain-containing (EHD) 1 and EHD3 recycling regulatory proteins. Axonal sorting and dynamic axonal transport of BACE1 are impaired in neurons when EHD function is compromised. EHD1/3 colocalize with BACE1 and APP β-C-terminal fragments *in vivo,* and loss of EHD1 or EHD3 expression leads to diminution of Aβ production in hippocampal neurons. Together, these findings characterize unidirectional endocytic BACE1 transport in neurons and identify a role for EHD family proteins in neuronal BACE1 trafficking and Aβ production. The results of this research show that somatodendritic and axonal BACE1 distribution in neurons is dynamically modulated by endocytic sorting in a trafficking pathway that requires EHD protein function. Endogenous EHD proteins colocalize with BACE1 in hippocampal mossy fibers, and loss of EHD1 or EHD3 expression in cultured neurons results in a significant decrease of Aβ secretion. These findings on EHD regulation of BACE1 trafficking and axonal targeting and Aβ production in neurons provide insights into presynaptic Aβ release and are highly relevant to abnormal presynaptic accumulation of BACE1 near amyloid plaques documented in Alzheimer's disease. (Buggia-Prévot et al., A function for EHD family proteins in unidirectional retrograde dendritic transport of BACE1 and Alzheimer's disease Aβ production, Cell Rep. 2013;5(6):1552-63).

The progressive accumulation of β-amyloid (Aβ) in senile plaques and in the cerebral vasculature is the hallmark of Alzheimer's disease and related disorders. Degradation of Aβ by specific proteolytic enzymes is an important process that regulates its levels in brain. Matrix metalloproteinase 2 (MMP2) was shown to be expressed in reactive astrocytes surrounding amyloid plaques and may contribute to Aβ degradation. Membrane type 1 (MT1) MMP, also known as MMP14, is the physiological activator for the zymogen pro-MMP2. It has been shown that in addition to MMP2, its activator MT1-MMP/MMP14 is also expressed in reactive astrocytes in regions with amyloid deposits in transgenic mice. Using a Cos-1 cell expression system, it was demonstrated that MT1-MMP/MMP14 can degrade exogenous Aβ40 and Aβ42. A purified soluble form of MT1-MMP/MMP14 degraded both soluble and fibrillar Aβ peptides in a time-dependent manner, yielding specific degradation products. Mass spectrometry analysis identified multiple MT1-MMP cleavage sites on soluble Aβ40 and Aβ42. MT1-MMP-mediated Aβ degradation was inhibited with the general MMP inhibitor GM6001 or the specific MT1-MMP/MMP14 inhibitor tissue inhibitor of metalloproteinases 2. Furthermore, in situ experiments showed that purified MT1-MMP/MMP14 degraded parenchymal fibrillar amyloid plaques that form in the brains of Aβ precursor protein transgenic mice. Together, these findings indicate that MT1-MMP possesses Aβ degrading activity *in vitro* (Liao MC and Van Nostrand WE. Degradation of soluble and fibrillar amyloid beta-protein by matrix metalloproteinase (MT1-MMP) in vitro. Biochemistry. 2010;49(6):1127-36).

As mentioned earlier, APP processing and Aβ production plays a central role in AD pathogenesis. Solid evidence indicate that ITM2B/BRI2 is an important physiological regulator of α-, β-, and γ-processing of APP. BRI2 physically associates with APP, and this interaction serves to suppress APP processing by secretases and the production of Aβ in vivo. Increasing ITM2B/BRI2 levels in the mouse brain by transgenic expression reduces APP cleavage by α- and β-secretase as well as amyloid plaque formation, while reducing ITM2B/BRI2 expression in cell lines by RNAi and by gene targeting in mice increases APP processing (Matsuda S et al. BRI2 Inhibits Amyloid β-Peptide Precursor Protein Processing by Interfering with the Docking of Secretases to the Substrate J Neurosci. 2008; 28(35): 8668-8676).

Horse chestnut seed contain various components, including i.a. escins (α-escins and β-escins), bioflavonoids (e.g. quercetine, kaempherol), anti-oxidants (proanthocianidin A₂) and coumarins (esculin abd fraxin) (Cesare R. Sirtori, "Aescin: Pharmacology, Pharmacokinetics and Therapeutic Profile", Pharmacological Research, Vol. 44, No. 3, 2001)). Horse chestnut extracts, for example horse chestnut seeds extracts, have been recognized by ethnopharmacological tradition as a remedy for fever, oedema and hemorrhoidal conditions. Clinical trials confirm that it may be also used in case of chronic venous insufficiency and capillary vessel fragility (EMEA Committee on Herbal Medicinal Products, Assessment Report on Aesculus hippocastanum L., semen, EMEA/HMPC/225304/2008, 1 (2009)).

β-escin is a mixture of triterpene saponins isolated from horse chestnut seeds (*Aesculus hippocastanum* L.). Historically, it was used to treat multiple diverse conditions, including bladder disorders, cough, diarrhea, dysmenorrhea and tinnitus. The anti-edematous, anti-inflammatory and venotonic properties of β-escin became particularly well established in ethnopharmacological tradition and currently they remain the most extensively clinically investigated effects of this plant-based drug. Major clinical indication for β-escin is treatment of chronic venous insufficiency.

There are no medications or supplements for Alzheimer's disease with evidence to support their use. None of the currently used treatments stop or reverse Alzheimer's disease progression, though some may temporarily improve symptoms. Available therapies may help with both cognitive and behavioral symptoms. Such therapies include e.g. use of donezepil, galantamine, memantine, rivastigmine and tacrine.

There is still a need for new treatment and/or prevention therapies of Alzheimer's disease. Thus, the aim of the present invention is to provide a new therapy, which could be used to treat and prevent the Alzheimer's disease.

### Subject of the invention

The subject of the invention is an agent selected from group comprising: horse chestnut extract, escins, their salts and derivatives, for use in treatment and/or prevention of Alzheimer's disease. In one embodiment, the agent of the invention is horse chestnut extract, β-escin or its salts.

In one embodiment, the agent used according to the present invention is contained in a pharmaceutical composition. Such composition may be preferably intended for oral, intravenous or transdermal administration.

In another embodiment, the agent used according to the present invention, may be administered 1-4 times per day.

In another embodiment, the agent used according to the present invention, may be co-administered with another active agent.

### Detailed description of the invention

The agents cited above (named herein as agents of the invention) share the analogous chemical structure (escin is a major component of the horse chestnut extract) as well as common pharmacological activity - the known in the prior art, and new one, shown in the experiments described in the attached examples.

The present invention is based on the finding that agents of the invention influence the concentration of four proteins involved in the pathogenesis of Alzheimer's disease: amyloid beta A4 protein (APP), a direct precursor of β-amyloid and integral membrane protein 2B (ITM2B or BRI2), which is a physiological suppressor of β-amyloid production, as well as Eps15 homology domain-containing protein (EHD1), and membrane type 1 matrix metalloproteinase 2 (MT1-MMP2 or MMP14) proteins.

The experiment illustrated in the Examples show that upon administration of β-escin, concentrations of APP, MPM14, and ITM2B proteins in Human Umbilical Vein Endothelial Cells (HUVEC) and peripheral blood mononuclear cells (PBMC) increases, while the concentration of EHD proteins in said cells decrease. This is a strong indication that the agents of the invention may be used as pharmaceuticals for treating and preventing Alzheimer's disease.

Although the experiments illustrated in the Examples have been conducted on Human Umbilical Vein Endothelial Cells (HUVEC) and peripheral blood mononuclear cells (PBMC), the obtained data constitute an evidence that said agents would be effective as pharmaceuticals for treating and preventing Alzheimer's disease. This assertion is based on prior art publications (e.g. Cesare R. Sirtori, "Aescin: Pharmacology, Pharmacokinetics and Therapeutic Profile", Pharmacological Research, Vol. 44, No. 3, 2001) describing that agents of the invention show systemic activity - their influence on the different pathologies is observed after oral administration. Therefore, the inventors believe that the APP, ITM2B, EHD, and MPM14 proteins concentrations would be influenced not only in the HUVEC and PBMC cells but also in other cell types, including those directly involved in the Alzheimer's disease pathology. This conclusion is further supported by research data, which confirm that endothelial function is involved in Alzheimer's disease pathology (Dede DS et al. Assessment of Endothelial Function in Alzheimer's Disease: Is Alzheimer's Disease a Vascular Disease? J Am Geriatr Soc. 2007;55(10):1613-7).

As mentioned above, agents of the invention may be used in treatment of the Alzheimer's disease, i.e. for the purpose of curing it, but also for the purpose of prevention (as prophylactic), control, amelioration, or alleviation of any symptom of the Alzheimer's disease.

The agents of the invention may be obtained by any method known in the prior art. Preferably, Horse chestnut extract may be obtained from horse chestnut seeds.

Certain agents used according to the invention may be used in free form or a salt form, especially pharmaceutically acceptable salt form. Pharmaceutically acceptable salts include, when appropriate, pharmaceutically acceptable base addition salts and acid addition salts, for example, metal salts, such as alkali and alkaline earth metal salts, ammonium salts, organic amine addition salts, etc. Examples of metal salts are alkali metal salts, such as lithium salt, sodium salt and potassium salt, alkaline earth metal salts such as magnesium salt and calcium salt, aluminum salt, and zinc salt. Examples of ammonium salts are ammonium salt and tetramethylammonium salt. Examples of organic amine addition salts are salts with morpholine and piperidine. Pharmaceutically acceptable salts include also acid addition salts, for example salts with inorganic acids such as hydrochloric, hydrobromic, hydroiodic, sulfuric and phosphoric acids, as well as organic acids.

Escins derivatives include synthetic and semi-synthetic compounds obtained based on the escins (e.g. α- or β- escin) or their components, such as saponins and aglycons.

The agents of the invention may be normally subject of administration by any route used in the prior art with respect to these agents.

Such routes include enteral (e.g. oral, etc.) or parenteral (e.g. intravenous, intramuscular, intradermal, intraperitoneal and subcutaneous injection or infusion, etc.) or topical (e.g. transdermal, etc.) administration. Preferred routes of administration include intravenous, oral, or transdermal routes.

The agents of the invention may be administered in any form used in the art in case of these agents. The administered doses of the agents of the invention may be varied so as to obtain an amount of the agent of the invention that is effective to achieve the desired therapeutic response for a particular patient, composition, and mode of administration. The selected dosage level will depend upon the activity of the particular agent, the route of administration, the severity of the condition being treated and the condition and prior medical history of the patient being treated. Conveniently, agents of the invention may be administered in form of pharmaceutical compositions.

Besides the agent of the invention, pharmaceutical composition may also contain at least one additional component, typical for the selected dosage form, which facilitates processing the compound of the invention into a pharmaceutical compositions. Such additionally components include diluents, excipients, carriers or other suitable auxiliary agent, e.g. fillers (such as sugars, cellulose preparations, calcium phosphates, etc.), binders (such as starch, gelatine, gum tragacanth, methylcellulose, hydroxypropylmethylcellulose, polivinylpyrrolidone, etc.), disintegrating agents (starch, cross-linked polyvinylpyrrolidone, alginic acid, etc), flow conditioners, lubricants (such as talc, stearic acid and its salts e.g. magnesium stearate, calcium stearate, etc.), glidants, sweeteners, fragrances, preservatives, stabilizers, wetting agents, emulsifiers, solubilizers, osmotic agents, buffers, antioxidants, etc. The pharmaceutical composition may also comprise other auxiliary agents, not mentioned above.

Pharmaceutical composition for parenteral administration by injection or infusion, for example for intravenous administration, suitably include pharmaceutically acceptable sterile aqueous or non-aqueous solutions, dispersions, suspensions or emulsions as well as sterile powders for reconstitution into sterile injectable solutions or dispersions prior to use. Such compositions may be presented in unit-dose or multi-dose containers, for example, sealed ampoules and vials.

Pharmaceutical compositions for oral administration include e.g. coated and uncoated tablets, pills, capsules, lozenges, as well as solutions, suspensions such as tinctures or elixirs, and powders, granulates and like, used to prepare them.

Examples of pharmaceutical composition useful for topical administration, for example transdermal, include lotions, emulsions, creams, gels, pastes, powders, foams, lipsticks, drops, sprays, solutions and suspensions.

The pharmaceutical compositions comprising the agents of the present invention may be manufactured in manner which is well known in the art. Accordingly, pharmaceutical compositions of the present inventions may be prepared by means of conventional mixing, granulating, dragee-making, dissolving and lyophilizing processes, etc. Selection of appropriate process will depend on the target dosage form.

The agents of the invention may be used in their typical amounts used in the prior art. As mentioned above, the dosages will depend on the administration route.

Horse chestnut extracts are usually administered by oral route, e.g. in form of tablets, capsules or tinctures. Amount of such extracts is usually calculated based on the β-escin content which, depending on the extract type, may be present in range of approx. 16-70%, for example 16 %, 17%, 18%, 20%, 30%, 40% 50%, 60% and 70%. Typically horse chestnut extract dosages administered orally are in range of 50-1500 mg/day, for example 50, 60, 100, 200, 250, 300, 400, 360, 600, 738, 824, 1500 mg/day, depending on the β-escin content. Examples of such dosages include: 200 mg, 300 mg, 600, 738 mg, 824 mg of Horse chestnut extract (containing 40 mg, 50 mg, 100 mg, 150 mg and 150 mg of escin, respectively). Horse chestnut extract may be also administered in a form of tincture, e.g. 2% tincture.

Horse chestnut extract dosages are usually administered up to 4 times a day.

Horse chestnut extract may be also administered intravenously in dosage from range approx. 1-10 mg/kg, for example in amount of 1, 2.5, 5 or 10 mg/kg.

For example, when administered orally, β-escin may be used in amount from range of 20-150 mg, for example 20 mg, 50 mg, 75, 100 mg, 120 mg or 150 mg may be administered in one dose or multiple doses e.g. 2-4 times a day.

When administered intravenously, β-escin may be used in amount from range 5-49 mg, for example 5, 10, 15, 20, 25, 30, 35, 40, 45 or 49 mg may be administered e.g. once or twice a day. Examples include dosages from range of 0.45-3.6 mg/kg of body weight.

In case of intraperitoneal administration, exemplary dosages of β-escin may be within a range of 1.4-2.8 mg/kg of body weight.

For example, for transdermal administration of β-escin, 1-5% gels may be used.

As mentioned above, the agents used according to the invention may be for example administered on a regimen of 1 to 4 times per day, preferably once or twice per day. The dosage regimen may be adjusted to provide the optimal therapeutic response.

Additionally, the compound of the invention may be administered alone or in combination with other active agents. Such additional active agents include e.g. other medicaments for treating symptoms of Alzheimer's disease or medicaments for treating conditions which are associated with or induced by this disease.

### Advantages of the invention

The results of the experiments illustrated in the Examples show that the use of the agents of the invention show a pharmacological activity which may be potentially beneficial in treatment and/or prevention of the Alzheimer's disease. In other words, the present invention provides a potentially effective therapy for Alzheimer's disease.

### Description of Figures

Fig. 1 shows the results of cell viability analysis
Fig. 2 shows the influence of β-escin on APP protein levels in HUVEC as measured by MRM technique
Fig. 3 shows the influence of β-escin on ITM2B protein levels in HUVEC as measured by MRM technique
Fig. 4 shows the influence of β-escin on MMP14 protein levels in HUVEC as measured by MRM technique
Fig. 5 shows the influence of β-escin on EHD protein levels in HUVEC as measured by MRM technique

### Examples

### Example 1.: Cell Viability

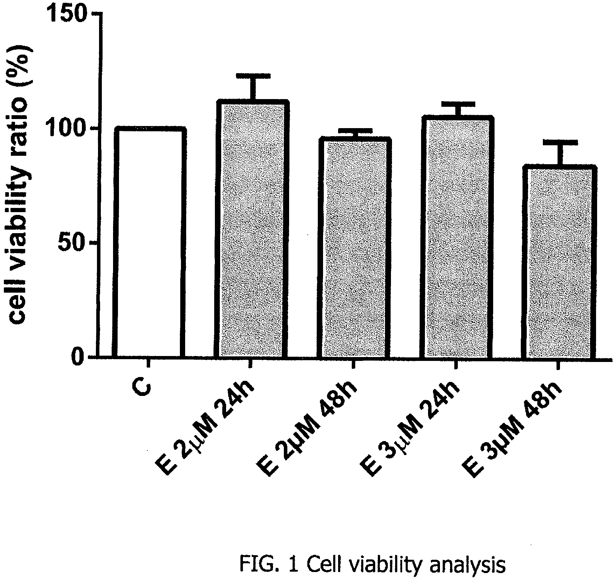

In order to evaluate the effect of β-escin on HUVEC viability we measured the number of viable cells. The Ultrasensitive Cell Proliferation Assay (Calbiochem, QIA128) measurig the ability of intracellular esterases to convert nonfluorescent substrate into a fluorescent product was used to evaluate the cell numbers. β-escin was shown not to adversely affect cell viability at 2 and 3 µM concentrations (Fig. 1, labels - E 2µM and E 3µM, respectively) after 24 and 48 hours of incubation.

### Example 2. β-escin effect on proteome of Human Umbilical Vein Endothelial Cells (HUVEC) and Peripheral Blood Mononuclear Cells (PBMC)

### Methodology

### Cell Culture

Human Umbilical Vein Endothelial Cells (HUVEC) were cultured in the endothelial cell basal growth medium-2 (EBM-2; Lonza, Basel, Switzerland) supplemented with endothelial growth supplement mix (EGM-2 SingleQuot Kit Supplements and Growth Factors, Lonza, Basel, Switzerland) under standard cell culture condition (37°C, 5% CO₂). Cells were harvested using Accutase (PAA Laboratories GmbH). The cells were treated with β-escin (2 and 3 µM, Nobilus Ent).

Peripheral Blood Mononuclear Cells (PBMC) separated from blood of young (aged 25-35 years) healthy donors, were cultured (40 mln/flask) in RPMI Medium (1x) + GlutaMAX (1x) (Gibco) supplemented with 10% Fetal Bovine Serum (Gibco), 1% Antibiotic/Antimycotic (PAA) and 1 mM HEPES (Sigma). Cells were treated for 24 h with β-escin (2 and 3 µM, Nobilus Ent). Cells in suspension were subjected to centrifugation (400xg, RT, 8 min.) The cell pellet was resuspended and washed in PBS (free of Ca and Mg cations, Dubelco) (400xg, RT, 8 min.). The pellet was stored at -80°C.

### Isobaric Tags for Relative and Absolute Quantitation (iTRAQ) analysis

HUVEC and PBMC were analyzed using iTRAQ. Three independent biological replicate sets were analyzed with three separate iTRAQ analyses. Each biological replicate was composed of a different donor cell pool composed of three unrelated donors. Each treatment sample was then analyzed with two technical replicates within the 8-plex iTRAQ assay (AB SCIEX, Framingham, MA, USA). Protein extracts (100 µg) were precipitated with 1 mL cold acetone overnight at -20°C and pelleted by centrifugation at 6000 x g for 10 min. Protein digestion and iTRAQ labeling was done as per manufacturer's instructions using the 8-plex iTRAQ assay and iTRAQ Reagent and Buffer Kits (AB SCIEX).

A Waters nanoACQUITY UPLC system was used for peptide separation prior to MS/MS analysis. Mobile phase A consisted of 0.1% FA and mobile phase B was ACN/0.1% FA. Twenty µL of the post IEF peptide extract was injected onto a reversed-phase trapping column (180 µm x 20 mm, C18, 5 µm, ACQUITY UPLC Symmetry, Waters) using mobile phase A. Peptides were transferred to a reversed-phase analytical column (75 µm x 250mm, nanoACQUITY UPLC BEH130 C18 Column, 1.7 µm, Waters) and separated using a 250 nL/min flow rate and a gradient from 3-33% mobile phase B over 150 minutes. The column was directly coupled to the ion source of an LTQ Orbitrap Velos (Thermo Fisher Scientific) which worked in the regime of data-dependent MS to MS/MS switch, with the following criteria: HCD fragmentation, one MS scan followed by a maximum of 5 MS/MS scans, 1.5kV capillary voltage and data acquired in positive polarity mode.

Data processing was performed as previously described (Bakun M et al., "Urine proteome of autosomal dominant polycystic kidney disease patients", Clin Proteomics. 2012 ;9(1):13). Briefly, Mascot Distiller (version 2.3.2.0, Matrix Science, London, UK) was used to pre-process MS/MS data with default value settings for iTRAQ labeling. This included the merging of redundant spectra and removal of noisy spectra. Next, a two-step database search procedure (Mikula et al. Comprehensive analysis of the palindromic motif TCTCGCGAGA: a regulatory element of the HNRNPK promoter J. DNA Res. 2010;17(4):245-60) was carried out. The differentially expressed proteins list with estimation of statistical significance of a single protein ratio was acquired using the in-house program Diffprot (Malinowska et al. Diffprot - software for non-parametric statistical analysis of differential proteomics data J Proteomics. 2012;75(13):4062-73.) as described previously (Bakun M et al. Urine proteome of autosomal dominant polycystic kidney disease patients. Clin. Proteomics. 2012 ;9(1):13).

### Sample preparation for MRM analysis

Fifty µg of protein extract was denatured in a final concentration of 1% NaDOC in 100 mM AmmBic. Disulphide bonds were next reduced with 2 µL of 50 mM TCEP (in 100mM AmmBic) for 1 hour at 60°C and alkylated with 2 µL of 100 mM iodoacetamide (Sigma-Aldrich) for 30 minutes at 37°C in the dark. Sequencing grade trypsin was added to obtain a 1:12.5 enzyme:protein ratio and digestion was carried out for 16 hours at 37°C.

To a volume corresponding to 25 µg of digested proteins a mixture of SIS peptides was added to give a an amount of SIS ranging from 50 to 500 fmol/µg of sample for each single SIS peptide and a final 0.33% formic acid concentration to precipitate the NaDOC detergent. NaDOC was removed by centrifugation at 14,000 x g for 10 minutes at room temperature. The supernatant was transferred to a polypropylene HPLC auto-sampler vial and analyzed by nano-LC-MRM.

MRM analysis was performed as described previously in Bakun et al. (2012) with minor modifications.

The optimization of peptide-specific MRM settings for the SID-MRM analysis was performed as previously described in Bakun et al. (Bakun et al., 2012) to generate the highest signal for each individual peptide and ion fragments.

For the SID-MRM analysis the quantity of endogenous peptide is reported as the Peak Area Ratio (PAR) which is the sum of the peak areas of all transitions for the endogenous peptide divided by the sum of the peak areas of transitions of its heavy standard. The addition of equivalent amounts of standard peptides to the analyzed samples and the calculation of PAR enables the normalization of natural peptide relative abundance between samples in terms of MS signal fluctuations and post-digestion sample processing differences.

LC-MRM-MS analysis was carried out with 1 µL of sample injected, corresponding to 1 µg of digested proteins, with blank (0.1% FA) runs in between every sample. All MRM data was processed using the Skyline Ver. 2.5 software with default values for peak integration and Savitzky-Golay peak smoothing. All integrated peaks were manually inspected to ensure correct peak detection and accurate integration. All peptides were targeted using 3 to 5 MRM ion pairs per peptide that were interference free.

### Statistical analysis

The results were expressed as arithmetic means and standard errors of the mean (SEM). Comparisons between the results obtained for the examined limbs and the results for control limbs were performed using the paired t-test (GraphPad Prism v.6.01). The differences at p < 0.05 were considered statistically significant.

### Results and discussion

### Differential proteome analysis of the β-escin-treated HUVEC and PBMC using iTRAQ

We applied iTRAQ method to discover proteins involved in β-escin induced cellular responses. We found that β-escin at 3 µM concentration affected expression of APP, ITM2B, MMP14 and EHD1, all playing important role in the pathogenesis of Alzheimer's disease. The protein amounts of APP, ITM2B and MMP14 were increased, while the cellular content of EHD1 was decreased.

### Validation of iTRAQ results using the MRM technique

To verify the iTRAQ findings we carried out multiple reaction monitoring (MRM) targeted MS analysis on selected proteins in HUVEC. The advantage of MRM analysis includes higher specificity and detection accuracy combined with the ability to process larger sample sets. The increased sample throughput allowed us to analyze the effect of β-escin at 2 and 3 µM concentrations, on cells obtained from at least four donors, each with two technical replicates.

The obtained results confirmed that the amount of all of the iTRAQ identified proteins were affected following β-escin treatment, indicating a strong inhibitory β-escin effect on amyloidogenesis.

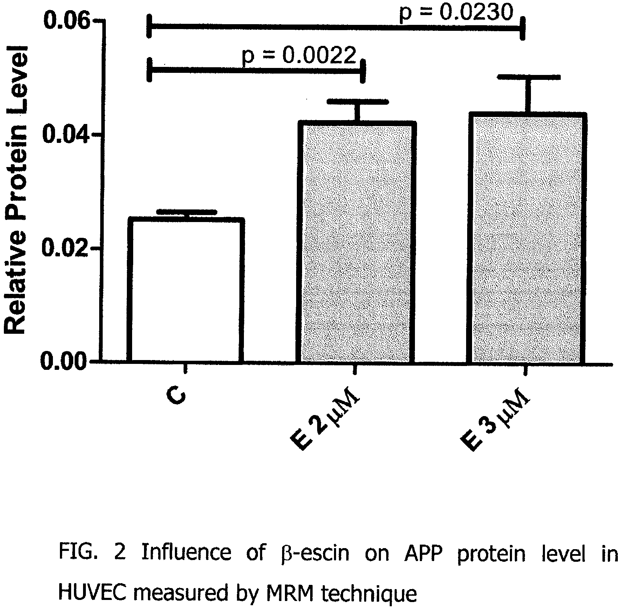

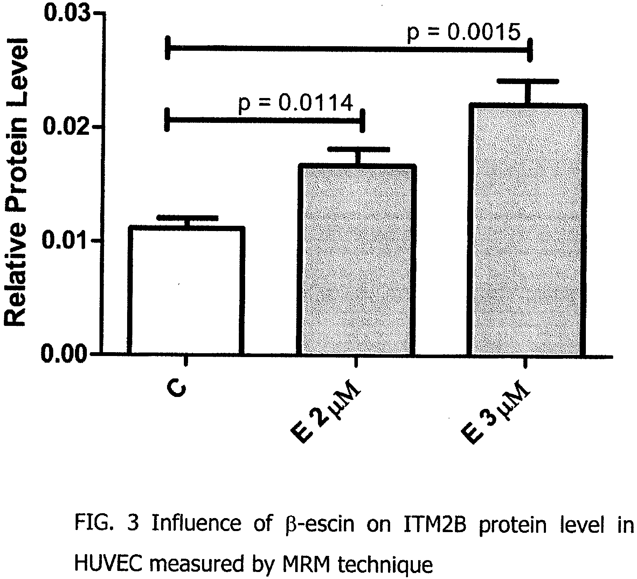

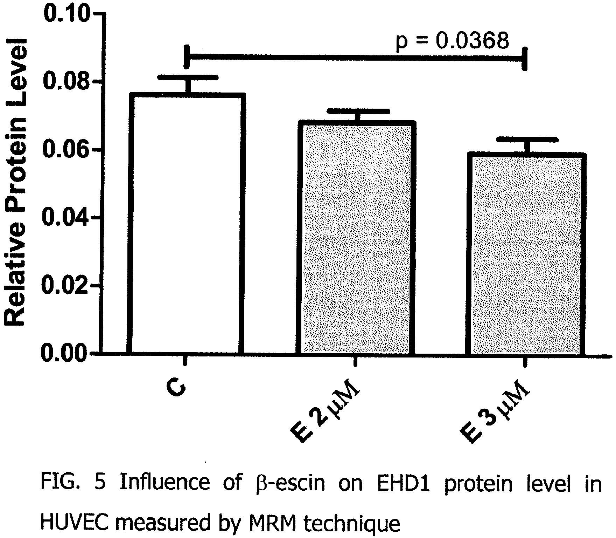

The results illustrated by Figures 2-5 (C - control, i.e. untreated cells, E 2µM - 2 µM β-escin concentration, E 3 µM - 3 µM β-escin concentration) show the following β-escin induced, concentration dependent changes in cellular protein profiles:
i) Fig.1 - an increase in APP, which may be at least in part due to the elevation in BRI2/ITM2B and reduction of APP processing,
ii) Fig. 2 - an increase in BRI2/ITM2B, a physiological suppressor of β-amyloid production,
iii) Fig. 3 - an increase in MT-1 MMP/MMP14, a molecule not only possessing Aβ degrading activity, but also physiologically activateing the pro-MMP2, which further contributes to Aβ degradation,

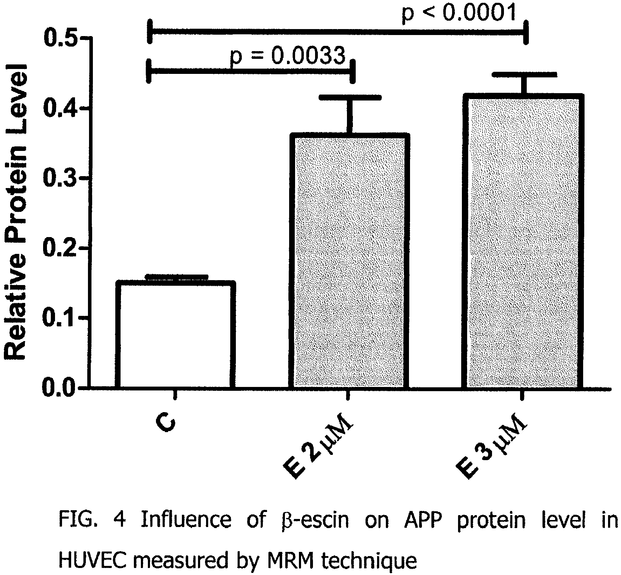

iv) Fig. 4 - a decrease in EHD1, a protein involved in β-secretase trafficking and Aβ production.

The results presented in the Figures 2-5 depict changes in cellular content of APP, MMP14, ITM2B and EHD proteins, all of which are significantly involved in Alzheimer's disease pathology. Therefore, the knowledge available in the art and above described results constitute a strong base for a conclusion that mechanisms of Alzheimer's disease could be influenced upon administration of agents of the invention. Consequently, the agents of the invention are potential medicaments for treatment and prevention of Alzheimer's disease.

## Claims

1. An agent selected from group comprising horse chestnut extract, escins, their salts and derivatives, for use in treatment and/or prevention of Alzheimer's disease.

2. The agent according to claim 1, wherein it is p-escin or its salt.

3. The agent according to claim 1, wherein it is a horse chestnut extract.

4. The agent according to any previous claims, wherein it is present in a pharmaceutical composition.

5. The agent according to claim 4, wherein the pharmaceutical composition is intended for oral, intravenous or transdermal administration.

6. The agent according to any of the previous claims, wherein it is administered 1-4 times per day.

7. The agent according to any of the previous claims, wherein it is co-administered with another active agent.
